(19) Europäisches Patentamt
European Patent Office
Office européen des brevets

(11) Publication number: 0 327 360 B1

(12) EUROPEAN PATENT SPECIFICATION

(45) Date of publication of patent specification: 06.07.94

(51) Int. Cl.⁵: **C12P 21/02**, C12N 15/25, C07K 13/00

(21) Application number: 89301007.4

(22) Date of filing: 02.02.89

(54) Novel polypeptides and DNA encoding said polypeptides.

(30) Priority: 03.02.88 JP 24613/88

(43) Date of publication of application:
09.08.89 Bulletin 89/32

(45) Publication of the grant of the patent:
06.07.94 Bulletin 94/27

(84) Designated Contracting States:
AT BE CH DE ES FR GB GR IT LI LU NL SE

(56) References cited:
EP-A- 0 188 920
EP-A- 0 200 986
EP-A- 0 237 073
EP-A- 0 237 967
EP-A- 0 259 160

PROC. NATL. ACAD. SCI. USA, vol. 84, July 1987, pp. 4572-4576, Washington, DC, US; B. MOSLEY et al.

THE JOURNAL OF BIOCHEMISTRY, vol. 104, no. 5, November 1988, pp. 837-840, Tokyo, JP; T. KAMOGASHIRA et al.

(73) Proprietor: **Dainippon Pharmaceutical Co., Ltd.**

25, Doshomachi 3-chome
Higashi-ku
Osaka-shi, Osaka 541(JP)

(72) Inventor: **Yamayoshi, Michiko**
**8-14, Nishimidorigaoka 3-chome**
**Toyonaka-shi Osaka-fu(JP)**
Inventor: **Kawashima, Hitoshi**
**47-27-205, Izumi-cho 2-chome**
**Suita-shi Osaka-fu(JP)**
Inventor: **Yamagishi, Junichi**
**309-10, Sugahara-cho**
**Nara-shi Nara-ken(JP)**
Inventor: **Kotani, Hirotada**
**1-11-403, Chayamadai 2-cho**
**Sakai-shi Osaka-fu(JP)**
Inventor: **Furuta, Ryuji**
**24-8, Fujimidai**
**Otsu-shi Shiga-ken(JP)**
Inventor: **Fukui, Toshikazu**
**4-8, Mishima 3-chome**
**Settsu-shi Osaka-fu(JP)**

(74) Representative: **Coleiro, Raymond et al**
**MEWBURN ELLIS**
**York House**
**23 Kingsway**
**London WC2B 6HP (GB)**

**Description**

This invention relates to novel polypeptides having a modified amino acid sequence of human interleukin 1 in which the N-terminal and C-terminal regions may optionally be deleted, that is, a certain amino acid residue(s) in said amino acid sequence being exchanged for other amino acid residue(s), and a DNA encoding the modified amino acid sequence, and a process for producing said polypeptides and said DNA.

Prior Art

Interleukin 1 is a physiologically active substance produced in various cells such as monocytic/macrophage cells. It is known that human interleukin 1 includes generally two types of polypeptides of α-type and β-type (cf. Furutani, Y. et at., Nucleic Acids Res., Vol. 14, 3167, 1986; Clark, B.D. et at., Nucleic acids Res., Vol. 14, 7897, 1986). It is also known that the interleukin 1 has activities such as promotion of proliferation of T and B lymphocytes, activation of lymphocyte, activation of macrophage, function of endogenous pyrogens, induction of production of prostaglandin $E_2$, and promotion of proliferation of fibroblast, and the like (cf. Oppenheim, J.J. et al., Immunology Today, Vol. 7, 45, 1986; Dinarello, C.A., Reviews of Infectious Diseases, Vol. 6, 51, 1984). Thus, interleukin 1 takes an important role in the control of immunological mechanism in living body and is expected to be clinically useful as a medicament.

Respective formulae of IL-1α and IL-1β polypeptides are given in Fig 5a of an article by March et al Nature, Vol. 315, 20 June, 1985, pages 641-647 at page 646.

The formulae of IL-1α and biologically active parts thereof (and of the DNA encoding them) are also given in EP-A-0188920 (see Tables 1-1, 2-1, 5 and 6 for the polypeptides and Tables 1-2, 2-2, 5 and 6 for the DNA), EP-A-0188864 (Figs IA-C) and EP-A-0200986 (Fig. 2).

From a comparison of the respective formulae of Table 5 of EP-A-0188920, Figs 1A-C of EP-A-0188864 and Fig 2 of EP-A-0200986 it can be seen that there are, apparently allelic variations between the respective amino acid sequences; note the respective amino acid residues at the 67 and 114 positions.

There are known, in particular, some derivatives of α-type human interleukin 1 polypeptide, such as human interleukin 1α polypeptide in which one to four amino acid residues at the C-terminus are deleted (see EP-A-0188920 supra) and human interleukin 1α polypeptide in which 36th amino acid (Asn) at the N-terminus is deamidated or exchanged for Asp (cf. Cameron, P.M. et al., J. Exp. Med., Vol. 164, 237, 1986; Wingfield, P. et al., Eur. J. Biochem., Vol. 165, 537, 1987).

Also known is a modified polypeptide of an α-type human interleukin 1 originating from any other gene in which the second amino acid residue (Ser) at the N-terminus is exchanged for Ala; indeed, the formula given by March et al supra is that of such a polypeptide.

As to β-type human interleukin 1 polypeptide, derivatives of the polypeptide are known in which one to three amino acid residues at the N-terminus and/or one to three amino acid residues at the C-terminus are deleted (cf. Mosley, B. et al, Proc. Natl. Acad. Sci. USA, Vol. 84, 4572, 1987).

Brief Description of the Invention

We have investigated the relationship between the chemical structure of human interleukin 1 and the biological activities thereof and the activities of modified polypeptides in which a certain portion of the amino acid sequence is exchanged for other amino acid residue(s) and have unexpectedly found that some modified polypeptides show almost no induction of the production of prostaglandin $E_2$ while they show activation of lymphocytes.

In particular, we have found that such a surprising change in the properties of IL-1α polypeptides may be obtained if, in a protein comprising at least a biologically active part of the IL-1α, the amino acid sequence is modified at least in a position which, in in Table 8, is arbitrarily numbered 263 in the formula given for precursor IL-1α, whereby the amino acid residue (shown in that position as Asp) is other than Asp, and is preferably Tyr.

For an IL-1α polypeptide, the amino acid residue at the 148 position of the said formula thereof may be Asn or Asp, especially Asp.

In a preferred IL-1α polypeptide at least the amino acid residues numbered from 1 to 112 inclusive are absent.

In addition to the abovementioned amino acid residues which are preferably absent from the IL-1α polypeptide, there may be additional deletions at the N-terminus and/or C-terminus.

3

The invention also provides, in particular, polypeptides of human interleukin 1α polypeptide in which a part of the amino acid sequence is exchanged for other amino acid residue and further a certain amino acid residue(s) at the N-terminus and/or the C-terminus are optionally deleted.

Thus, for such an IL-1α polypeptide (preferably without the abovementioned 1-112 amino acid sequence) one to fourteen amino acids may be deleted from the N-terminus and/or one to four may be deleted from the C-terminus.

As is clearly apparent to the man skilled in the art, the numbering in the abovementioned formulae is purely arbitrary and may vary, for example, if certain amino acid residues were added or deleted. The invention is not limited to modification of a specifically numbered amino acid residue in a specific formula.

Thus the invention includes any polypeptide modified as described above and possessing the biological activity of the IL-1α so modified, for example polypeptides additionally modified to provide shorter or longer variants or alleles such as those described in EP-A-0188920, EP-A-0188864 and EP-A-0200986 supra.

In addition to the modified polypeptides described above, the invention also provides DNA encoding such polypeptides, and processes for producing such DNA and polypeptides, expression vectors containing DNA capable of expressing the modified polypeptides and arranged for expression thereof in a suitable host, recombinant host organisms transformed with such DNA so as to be capable of expression thereof, and medicaments comprising the polypeptides.

Brief Description of the Drawings

Fig. 1 shows steps of the construction of expression plasmid pHTN55(141) of this invention, wherein the synthetic DNA adaptors [C], [D] and [E] are the chemically synthesized oligonucleotide adaptors as described in Example 3.

Fig. 2 shows steps of the construction of expression plasmid pHIPH383a of this invention, wherein the synthetic DNA adaptors [F] and [G] are the chemically synthesized oligonucleotide adaptors as described in Reference Example 1.

Fig. 3 shows steps of the construction of expression plasmid pHIP312EC of this invention, wherein the synthetic DNA linker [H] is the chemically synthesized oligonucleotide linker as described in Reference Example 2.

Detailed Description of the Invention

The modified polypeptide of this invention includes a human interleukin 1α polypeptide in which partial amino acid residues are exchanged for other amino acid residue(s) whereby the polypeptide is (a) essentially incapable of inducing the production of prostaglandin $E_2$ and (b) capable of activating lymphocytes, i.e. a polypeptide having such biological activity and having an amino acid sequence of the formula [I] in Table 1 in which one to fourteen amino acid residues or peptide at the N-terminus and/or one to four amino acid residues or peptide at the C-terminus are optionally deleted.

4

**Table 1**

```
Ser   W  Pro Phe Ser Phe Leu Ser Asn Val

Lys Tyr Asn Phe Met Arg Ile Ile Lys Tyr

Glu Phe Ile Leu Asn Asp Ala Leu Asn Gln

Ser Ile Ile Arg Ala  X  Asp Gln Tyr Leu

Thr Ala Ala Ala Leu His Asn Leu Asp Glu

Ala Val Lys Phe Asp Met Gly Ala Tyr Lys

Ser Ser Lys Asp Asp Ala Lys Ile Thr Val

Ile Leu Arg Ile Ser Lys Thr Gln Leu Tyr

Val Thr Ala Gln Asp Glu Asp Gln Pro Val

Leu Leu Lys Glu Met Pro Glu Ile Pro Lys

Thr Ile Thr Gly Ser Glu Thr Asn Leu Leu

Phe Phe Trp Glu Thr His Gly Thr Lys Asn

Tyr Phe Thr Ser Val Ala His Pro Asn Leu

Phe Ile Ala Thr Lys Gln Asp Tyr Trp Val

Cys Leu Ala Gly Gly Pro Pro Ser Ile Thr

 Y  Phe Gln Ile Leu Glu Asn Gln Ala
```

Formula [I]

in which W means Ser or Ala, X means Asn or Asp, and Y means an amino acid residue other than Asp.

The preferred polypeptide of this invention is the polypeptide of the formula [I] in Table 1 wherein W is Ser, X is Asn, and Y is Tyr, and the polypeptide having the amino acid sequence of the formula [III] in Table 3.

Table 3

```
Met Arg Ile Ile Lys Tyr Glu Phe Ile Leu

Asn Asp Ala Leu Asn Gln Ser Ile Ile Arg

Ala  X  Asp Gln Tyr Leu Thr Ala Ala Ala

Leu His Asn Leu Asp Glu Ala Val Lys Phe

Asp Met Gly Ala Tyr Lys Ser Ser Lys Asp

Asp Ala Lys Ile Thr Val Ile Leu Arg Ile

Ser Lys Thr Gln Leu Tyr Val Thr Ala Gln

Asp Glu Asp Gln Pro Val Leu Leu Lys Glu

Met Pro Glu Ile Pro Lys Thr Ile Thr Gly

Ser Glu Thr Asn Leu Leu Phe Phe Trp Glu

Thr His Gly Thr Lys Asn Tyr Phe Thr Ser

Val Ala His Pro Asn Leu Phe Ile Ala Thr

Lys Gln Asp Tyr Trp Val Cys Leu Ala Gly

Gly Pro Pro Ser Ile Thr  Y  Phe Gln Ile

Leu
```

Formula [III]

wherein X is Asn or Asp and Y is Tyr.

This invention also provides derivatives of the above polypeptides, which derivatives are formed by using the functional groups on the polypeptide chain, the N-terminal amino group, or the C-terminal carboxyl group, for example, esters of the carboxyl group with an aliphatic alcohol, acid amide derivatives with primary or secondary amines, N-acyl derivatives on the amino group, O-acyl derivative on the hydroxyl group, hydrolysate of carbamoyl group or modified product with polyethylene glycol. The derivatives include also salts of the carboxyl or amino group in the polypeptides with bases or acids (e.g. potassium hydroxide, arginine, caffein, procaine, hydrochloric acid, gluconic acid, etc.).

The polypeptide of this invention may optionally form a higher molecular weight compound by an intramolecular S-S bonding thereof. Such higher molecular weight compounds are also included in the polypeptide of this invention. Moreover, the polypeptide of this invention may include such a polypeptide in which Met is additionally present at the N-terminus which is produced depending on the kinds of cells or conditions for production thereof.

This invention provides also DNAs encoding the polypeptides of this invention. The DNAs include, for example, a DNA encoding the polypeptide having an amino acid sequence of the formula [IV] in Table 4 (hereinafter, said polypeptide being referred to as "TN-55 polypeptide"), i.e. a DNA having a nucleotide sequence of the formula [A] in Table 5, and degeneration thereof.

## Table 4

Ser Ser Pro Phe Ser Phe Leu Ser Asn Val

Lys Tyr Asn Phe Met Arg Ile Ile Lys Tyr

Glu Phe Ile Leu Asn Asp Ala Leu Asn Gln

Ser Ile Ile Arg Ala Asn Asp Gln Tyr Leu

Thr Ala Ala Ala Leu His Asn Leu Asp Glu

Ala Val Lys Phe Asp Met Gly Ala Tyr Lys

Ser Ser Lys Asp Asp Ala Lys Ile Thr Val

Ile Leu Arg Ile Ser Lys Thr Gln Leu Tyr

Val Thr Ala Gln Asp Glu Asp Gln Pro Val

Leu Leu Lys Glu Met Pro Glu Ile Pro Lys

Thr Ile Thr Gly Ser Glu Thr Asn Leu Leu

Phe Phe Trp Glu Thr His Gly Thr Lys Asn

Tyr Phe Thr Ser Val Ala His Pro Asn Leu

Phe Ile Ala Thr Lys Gln Asp Tyr Trp Val

Cys Leu Ala Gly Gly Pro Pro Ser Ile Thr

Tyr Phe Gln Ile Leu Glu Asn Gln Ala

Formula [IV]

## Table 5

5'-TCA TCA CCT TTT AGC TTC CTG AGC AAT GTG

AAA TAC AAC TTT ATG AGG ATC ATC AAA TAC

GAA TTC ATC CTG AAT GAC GCC CTC AAT CAA

AGT ATA ATT CGA GCC AAT GAT CAG TAC CTC

ACG GCT GCT GCA TTA CAT AAT CTG GAT GAA

```
GCA GTG AAA TTT GAC ATG GGT GCT TAT AAG

TCA TCA AAG GAT GAT GCT AAA ATT ACC GTG

ATT CTA AGA ATC TCA AAA ACT CAA TTG TAT

GTG ACT GCC CAA GAT GAA GAC CAA CCA GTG

CTG CTG AAG GAG ATG CCT GAG ATA CCC AAA

ACC ATC ACA GGT AGT GAG ACC AAC CTC CTC

TTC TTC TGG GAA ACT CAC GGC ACT AAG AAC

TAT TTC ACA TCA GTT GCC CAT CCA AAC TTG

TTT ATT GCC ACA AAG CAA GAC TAC TGG GTG

TGC TTG GCA GGG GGG CCA CCC TCT ATC ACT

TAC TTT CAG ATA CTG GAA AAC CAG GCG-3'
```

Formula [A]

The DNA encoding the polypeptide of this invention can be obtained by preparing a DNA encoding the known human interleukin 1 polypeptide in which the amino acid residue(s) or peptide at the N-terminus and/or the C-terminus may optionally be deleted by a known method, for example by a method disclosed in the above-mentioned European Patent Publication 0188920 and then varying it partially by a method of Wang et al. (cf. Wang, A.M. et al., Science, Vol. 224, 1431, 1984) or by a method of Kunkel et al. (cf. Kunkel, T.A. et al., Methods in Enzymol., Vol. 154, 367, 1987), or alternatively by isolating an appropriate DNA segment with an appropriate restriction enzyme and combining it with a synthetic oligodeoxynucleotide adaptor in which the desired part(s) of the nucleotide sequence is(are) artificially modified.

These DNAs are inserted into an appropriate expression vector for transformation in the desired sequence by a known method, transforming a host with the expression vector, and cultivating the resulting transformant, by which the desired polypeptide can be produced. For instance, an expression vector for the production of the polypeptide of this invention can be constructed by preparing a DNA fragment containing the nucleotide sequence encoding the polypeptide of this invention in which the translation initiation codon ATG is added to the 5'-terminus and a termination codon is added to the 3'-terminus, ligating the resultant DNA fragment with an appropriate promoter and the Shine-Dalgarno (SD) sequence, and inserting the resultant into a vector. The promoter includes, for example, lac, trp, tac, phoS, phoA, PL, SV40 early stage promoter, and the like. The vector includes, for example, plasmids (e.g. pBR322 plasmid, etc.), phages (e.g. λ phage derivative, etc.), viruses (e.g. SV40 etc.), runaway plasmid, and the like.

The expression vector for the production of the polypeptide of this invention is then introduced into an appropriate host (e.g. Escherichia coli) by a method of Cohen et al. (cf. Cohen, S.N., et al., Proc. Natl. Acad. Sci., USA, Vol. 69, 2110, 1972) to give a transformant. The transformant is cultivated under suitable cultivation conditions to give the desired polypeptide or a polypeptide with Met at the N-terminus. The host cells in the culture broth thus obtained are destroyed, for example, by lysozyme digestion and freezing and thawing or sonication, or by using a French press, and then centrifuged or filtered to collect the extract of the polypeptide of this invention.

The polypeptide thus obtained can be purified by conventional purifying methods for proteins, for example, by ultrafiltration, dialysis, electrophoresis, salting out, gel filtration, ion exchange chromatography, affinity chromatography with an antibody column, and the like.

The derivatives of the polypeptide of this invention can also be prepared by chemically modifying the polypeptide obtained above.

As to TN-55 polypeptide having the amino acid sequence shown in Table 4, which is the representative example of the polypeptide of this invention, there are shown the various properties thereof below.

Experiment 1

Physicochemical properties of TN-55 polypeptide:

The molecular weight and isolectric point were measured as follows.

The molecular weight was 18 ± 1 kD when measured by sodium dodecyl sulfate (SDS)-polyacrylamide gel electrophoresis, and the isoelectric point was 5.7 ± 0.2 when measured by an isoelectric point electrophoresis.

The partial amino acid sequence of TN-55 polypeptide was determined as follows.

The polypeptide was previously treated with 4-vinylpyridine and then subjected to an enzymolysis with lysylendopeptidase (manufactured by Wako Junyaku, Japan) in the presence of urea at 35°C overnight and the resulting peptide fragment was isolated by high performance liquid chromatography with SynChro Pack RP-P 300 column (manufactured by SynChrom Co., USA). As to the peptide fragment thus obtained and the polypeptide not treated with a proteinase, the amino acid sequence was determined with an automatic amino acid sequencer (470A Protein Sequencer, manufactured by Applied Biosystems Co., USA).

The amino acid sequence of the proteinase-untreated TN-55 polypeptide from the N-terminus to 40th amino acid residue was determined as follows, and was completely identical with the amino acid sequence shown in Table 4.

```
Ser Ser Pro Phe Ser Phe Leu Ser Asn Val

Lys Tyr Asn Phe Met Arg Ile Ile Lys Tyr

Glu Phe Ile Leu Asn Asp Ala Leu Asn Gln

Ser Ile Ile Arg Ala Asn Asp Gln Tyr Leu
```

On the other hand, the two kinds of peptide fragments obtained by the above treatment with a proteinase (designated LP-10 fragment and LP-12 fragment, respectively) showed the following amino acid sequences, respectively.

Amino acid sequence of LP-10 fragment:

```
Thr Ile Thr Gly Ser Glu Thr Asn Leu Leu



Phe Phe Trp Glu Thr His Gly Thr Lys
```

Amino acid sequence of LP-12 fragment:

```
Gln Asp Tyr Trp Val Cys Leu Ala Gly Gly

Pro Pro Ser Ile Thr Tyr Phe Gln Ile Leu

Glu Asn Gln Ala
```

These amino acid sequences of LP-10 fragment and LP-12 fragment were identical with the amino acid sequences of the 101st to 119th region and the 136th to 159th region at the N-terminus in Table 4.

Experiment 2

Biological activities of TN-55 polypeptide:

(1) Activation of lymphocyte

The activation of lymphocyte was measured by the following method. That is, an aqueous solution of TN-55 polypeptide was diluted with a tissue culture medium (RPMI-1640 medium, manufactured by Flow Labs., USA) which contained 5 % bovine fetal serum. The diluted solution (each 50 $\mu$l) was poured into wells of a flat bottomed plate with 96 wells, and to each well was added phytohemaglutinine-P (manufactured by Difco Labs., USA, concentration 20 $\mu$g/ml) (each 50 $\mu$l) and thereto was further added a suspension of thymic cells of C3H/He mouse (200 x $10^4$ cell/ml) (each 100 $\mu$l), which was cultivated in 5 % $CO_2$-air at 37°C for 3 days. About 18 hours before completion of the cultivation, $^3$H-thymidine (1 $\mu$Ci) was added, and the uptake amount of $^3$H-thymidine in cells was measured. In the negative control, RPMI-1640 medium containing 5 % bovine fetal serum was added instead of test sample, and the uptake amount of $^3$H-thymidine in the cultivated thymic cells of mouse was measured likewise. The results are shown in Table 6. As is clear from the results, the TN-55 polypeptide shows potent activity for activation of lymphocyte.

Table 6

| Final dilution fold of TN-55 polypeptide | Uptake amount of $^3$H-thymidine |
|---|---|
| 5,000 | 94,171 cpm |
| 20,000 | 95,283 |
| 50,000 | 83,579 |
| 200,000 | 69,471 |
| 500,000 | 65,143 |
| 2,000,000 | 39,050 |
| 5,000,000 | 16,630 |
| 20,000,000 | 9,952 |
| 50,000,000 | 6,311 |
| Negative control | 6,740 |

(2) Capacity of promotion of production of prostaglandin $E_2$:

As to the aqueous solution of TN-55 polypeptide used in the above measurement of activation of lymphocyte, the capacity of induction of production of prostaglandin $E_2$ of TN-55 polypeptide was measured by using as a target cell human osleosarcoma MG-63 cells (American Type Cell Collection CRL 1427) as follows. That is, an aqueous solution of TN-55 polypeptide was diluted with a tissue culture medium MEM Earle (manufactured by Flow Labs.) which contained 10 % bovine fetal serum, non-essential amino acids and sodium pyruvate. Each diluted solution (each 100 $\mu$l) was poured into wells of a flat bottomed plate with 24 wells, and to each well was added a cell suspension containing MG-63 cells (about 2 x $10^4$ cells) (each 400 $\mu$l), which was cultivated in 5 % $CO_2$-air at 37°C for 48 hours. After completion of the cultivation, the conditioned medium was separated, and the content of prostaglandin $E_2$ in the conditioned medium was measured with RIA kit (manufactured by E.I. duPont de Nemours & Co., USA). In the negative control, the above culture medium was added instead of the test solution, and the content of prostaglandin $E_2$ in the conditioned medium of MG-63 cells was measured likewise.

The results are shown in Table 7. As is clear from the results, this polypeptide shows almost no capacity of induction of production of prostaglandin $E_2$.

Table 7

| Final dilution fold of TN-55 polypeptide | Content of produced prostaglandin $E_2$ (ng/ml) |
|---|---|
| 500 | 0.36 |
| 5,000 | 0.30 |
| 50,000 | 0.18 |
| 500,000 | 0.18 |
| 5,000,000 | 0.21 |
| 50,000,000 | 0.18 |
| Negative control | 0.18 |

As is clear from the above experimental results, TN-55 polypeptide shows almost no capacity of induction of production of prostaglandin $E_2$ even in an amount in which it exhibits potent activation of lymphocyte, and hence, this product is a polypeptide having very characteristic properties.

For simplification of the description, the following abbreviations are used in the present specification and claims.

A: adenine
C: cytosine
G: guanine
T: thymine
DNA: deoxyribonucleic acid
cDNA: complementary DNA
RNA: ribonucleic acid
mRNA: messenger RNA
ATP: adenosine triphosphate
dATP: deoxyadenosine triphosphate
dCTP deoxycytidine triphosphate
dGTP deoxyguanosine triphosphate
dTTP deoxythymidine triphosphate
bp: base pairs
kbp: kilobase pairs
kD: kilodaltons
SDS sodium laurylsulfate

Embodiments of this invention are illustrated in more detail by the following Examples and Reference Examples.

Example 1

Production of TN-55 polypeptide:

TN-55 polypeptide having an amino acid sequence of the formula [IV] in Table 4 was produced by the following method.

(1) Construction of expression plasmid for transformation:

The recombinant plasmid pHIP312EC in Reference Example 2 was used as the starting material. The codon (GAC) encoding the 9th amino acid (Asp) at the C-terminus of human interleukin 1 polypeptide in said starting plasmid DNA was exchanged for the codon (TAC) corresponding to tyrosine by a site-specific mutation method similar to the method of Kunkel et al. (cf. Kunkel, T.A. et al., Methods in Enzymol., Vol. 154, 367, 1987), by which the expression plasmid for production of TN-55 polypeptide (pHTN55) was constructed.

The detail of the construction method is as follows.

The site-specific mutation was carried out by using MUTA-GENE In Vitro Mutagenesis Kit (manufactured by Bio-Rad Labs., USA). The plasmid pHIP312EC was digested with restriction enzymes PvuII and HindIII to isolate a DNA fragment containing a human interleukin 1 polypeptide-coding region and an E. coli

11

EP 0 327 360 B1

tryptophane operon promoter region. This DNA fragment was inserted into M13mp19 vector between the restriction enzyme HindIII recognition site and the restriction enzyme HincII recognition site. E. coli JM105 strain was infected with this recombinant DNA and then cultivated, and the resulting phage was collected. E. coli JM105 strain was infected with this phage and cultivated in 2xTY medium [composition: 1.6 % tryptone, 1 % yeast extract, 0.5 % sodium chloride] which contained uridine (1 $\mu$g/ml) and chloramphenicol (20 $\mu$g/ml) at 37°C for 5 hours, and the phage DNA was isolated from the culture supernatant, and thereby there was obtained the phage DNA of about 2 $\mu$g per 10 ml of the culture supernatant.

Separately, an oligodeoxyribonucleotide having a nucleotide sequence of the following formula [B] was chemically synthetized by a conventional method.

5′-CTATCACTTACTTTCA-3′     [B]

This nucleotide sequence was identical with the sequence of 779th to 794th bases in the nucleotide sequence in Table 8 except the 787th base. This chemically synthesized DNA was designated as "mutated primer".

Phosphoric group was added to the mutated primer at 5′-terminus thereof, and the primer was incubated with the above-prepared phage DNA in an anneal buffer [composition: 20 mM Tris-HCl buffer (pH 7.4) containing 2 mM magnesium chloride and 50 mM sodium chloride] at 70°C for 10 minutes, and the mixture was gradually cooled to 30°C at a lowering rate of 1°C/minute, by which the primer was ligated with the phage DNA. The phage DNA was treated with T4 DNA polymerase and T4 DNA ligase in the presence of dGTP, dATP, dCTP, dTTP and ATP etc. whereby DNA complementary to the phase DNA was synthesized to form a circular double-stranded DNA. E. coli JM105 strain was infected with this cyclic double-stranded DNA, and each clone was cultivated and from the cultivated cells there was isolated a replicative mutated form of double-stranded DNA.

The nucleotide sequence of the DNA including the protein coding region was determined by using single-stranded DNA isolated from the culture supernatant by which it was confirmed that it had a nucleotide sequence encoding TN-55 polypeptide.

By digesting the replicative form of double-stranded DNA with restriction enzymes HpaI and XhoI, there was taken out a DNA fragment containing the protein coding region. This DNA fragment is designated as "TN-55 DNA fragment".

Separately, by digesting the recombinant plasmid pHIPH383a in Reference Example 1 with restriction enzymes HpaI and XhoI, there was taken out a large DNA fragment containing ampicillin resistance gene and replication origin. This DNA fragment was ligated by T4 DNA ligase with the above TN-55 DNA fragment to construct expression plasmid "pHTN55".

This expression plasmid pHTN55 was introduced into E coli HB101 strain to prepare a transformant by the following method. That is, E. coli HB101 strain was inoculated to LB medium [composition: 1 % tryptone, 0.5 % yeast extract, 1 % sodium chloride (pH 7.5)] and incubated at 30°C overnight. The cell suspension (1 ml) was inoculated to LB medium (100 ml) and cultivated at 30°C until the turbidity (absorbance at 600 nm) became about 0.6. The cultivated medium was allowed to stand in ice water for 30 minutes and centrifuted to separate cells. The cells were re-suspended in 50 mM calcium chloride solution (50 ml) and the mixture was allowed to stand in ice water for 60 minutes and centrifuged to separate cells. The cells were suspended in 50 mM calcium chloride solution containing 20 % glycerin (10 ml). To the suspension was added the above expression plasmid pHTN55, and the mixture was incubated in ice water for 20 minutes and further at room temperature for 10 minutes, and thereto was added LB medium. The mixture was cultivated with shaking at 37°C for 60 minutes. An aliquot of the resulting cell suspension was spread on LB agar plate (agar concentration 1.5 %) containing ampicillin (25 $\mu$g/ml) and cultivated at 37°C overnight to obtain ampicillin resistant clone. This ampicillin resistant clone, i.e. transformant, was designated as "HB101/pHTN55" and used for producing TN-55 polypeptide.


(2) Production of TN-55 polypeptide


The HB101/pHTN55 for producing TN-55 polypeptide as obtained in the above (1) was cultivated in LB medium at 37°C overnight. The resulting cell suspension was inoculated in about 100-fold volume of a nutrient medium [composition: 1.5 % disodium phosphate•12 hydrate, 0.3 % monopotassium phosphate, 0.1 % ammonium chloride, 2 mg/l vitamin $B_1$, 0.5 % casamino acid, 2 mM magnesium sulfate, 0.1 mM calcium chloride, 1 % tryptone, 0.5 % yeast extract, 1 % sodium chloride, 0.4 % glycerine] and thereto was added indol-3-acrylic acid at a final concentration of 20 $\mu$g/ml, and the mixture was cultivated for 28 hours. The cells were collected by centrifugation and suspended in 50 mM Tris-HCl buffer (pH 8.0) containing 0.1 % lysozyme and 30 mM sodium chloride. The mixture was allowed to stand in ice water for 30 minutes and the cells were destroyed by repeating freezing in dry ice/ethanol bath and thawing at 37°C. To the mixture

12

was added 1/50 volume of 10 % polyethyleneimine, and the mixture was allowed to stand and centrifuged to remove cell debris etc. To the extract thus obtained was added ammonium sulfate until 70 % saturation, and the mixture was allowed to stand and centrifuged to collect the precipitates. The precipitates were dissolved in distilled water, dialyzed against 5 mM phosphate buffered saline (pH 6.5) [hereinafter referred to "PBS"], and then subjected to gel filtration with Sephacryl® S-200 column (manufactured by Pharmacia, Sweden). From a fraction corresponding to the molecular weight of about 15 to 20 kD, a solution containing TN-55 polypeptide was collected and charged to DEAE-Sepharose® CL-6B column (Pharmacia) which was previously equilibrated with 5 mM phosphate buffer (pH 6.5) (hereinafter referred to as "PB"), and thereafter the column was washed with PB. The TN-55 polypeptide adsorbed to the column was eluted with PB having 0 to 0.5 M concentration gradient of sodium chloride. As a result, polypeptide having a molecular weight identical with the theoretical molecular weight of TN-55 polypeptide was eluted in two fractions. The fraction eluted with a lower concentration of sodium chloride was designated as "TN-55(a) polypeptide fraction", and the fraction eluted with a higher concentration of sodium chloride was designated as "TN-55-(b) polypeptide fraction".

The TN-55(a) polypeptide fraction was dialyzed against 5 mM phosphate buffer (pH 5.7) (hereinafter referred to as "PB5"), charged to S-Sepharose® First Flow column (Pharmacia) which was previously equilibrated with PB5, and then eluted with PB5 having 0 to 0.25 M concentration gradient of sodium chloride. The fraction of TN-55(a) polypeptide was collected and concentrated by ultrafiltration, and thereafter, purified by gel filtration with Toyopearl® HW-55 column (manufactured by TOSOH, Japan) to obtain a highly purified product which had no impurity when analyzed with SDS-polyacrylamide gel electrophoresis.

The purified polypeptide obtained from TN-55(a) polypeptide fraction was subjected to the above-mentioned Experiments (1) and (2).

By the experiments, it was confirmed that the polypeptide was TN-55 polypeptide.

Example 2

Production of deamidated product of TN-55 polypeptide:

By the following method there was isolated a modified polypeptide, i.e. TN-55 polypeptide in which 36th amino acid at the N-terminus was exchanged for Asp (hereinafter referred to as "TN-55Asp polypeptide").

From the TN-55(b) polypeptide fraction eluted with a higher concentration of sodium chloride by DEAE-Sepharose® CL-6B column chromatography in Example 1-(2), there was isolated a polypeptide in the same manner as described in Example 1-(2). That is, a highly purified product was obtained by S-Sepharose® First Flow column chromatography and gel filtration with Toyopearl® HW-55 column.

As to the purified polypeptide from the TN-55(b) polypeptide fraction, the amino acid sequence was sequentially analyzed from the N-terminus by Edman degradation method. As a result, the polypeptide had the following amino acid sequence of from the N-terminus to the 40th amino acid.

```
Ser Ser Pro Phe Ser Phe Leu Ser Asn Val

Lys Tyr Asn Phe Met Arg Ile Ile Lys Tyr

Glu Phe Ile Leu Asn Asp Ala Leu Asn Gln

Ser Ile Ile Arg Ala Asp Asp Gln Tyr Leu
```

Thus, the 36th amino acid from the N-terminus was identified as Asp. Accordingly, it was concluded that the purified polypeptide was TN-55Asp polypeptide. This TN-55Asp polypeptide showed an isoelectric point of 5.4 ± 0.2 when measured by an isolectric point electrophoresis.

Example 3

Production of TN-55(141) polypeptide:

By the following method, there was isolated a polypeptide having an amino acid sequence of the formula [III] in Table 3 (wherein X is Asn and Y is Tyr), i.e. TN-55 polypeptide in which a peptide having fourteen amino acid residues at the N-terminus and a peptide having four amino acid residues at the C-terminus were deleted [hereinafter referred to as "TN-55(141) polypeptide"].

(1) Construction of expression plasmid:

The recombinant plasmid pHL4 inserted with a cloned cDNA encoding human interleukin 1 precursor polypeptide as prepared by the method disclosed in European Patent Publication 0188920 was digested with a restiction enzyme PstI to cut out the cDNA region. The human interleukin 1 precursor polypeptide encoded by this cDNA has the amino acid sequence and nucleotide sequence as shown in Table 8. The cloned cDNA was digested with restriction enzymes EcoRI and Sau96I to isolate DNA fragment corresponding to the 398th to 769th bases in the nucleotide sequence shown in Table 8.

This DNA was ligated by T4 DNA ligase with two kinds of chemically synthesized oligonucleotide adaptors of the following Formulae [C] and [D].

The chemically synthesized oligonucleotide adaptors [C] and [D] have the following nucleotide sequences:

```
5'-AAATTATGAGGATCATCAAATACG
    3'-TAATACTCCTAGTAGTTTATGCTTAA                    [C]
```

and

```
5'-GGCCACCCTCTATCACTTACTTTCAGATACTGTGATGACTCGA
    3'-GTGGGAGATAGTGAATGAAAGTCTATGACACTACTGAGCTCTAG      [D]
```

The DNA fragment thus obtained was designated as "IL1(141) fragment".

Expression vector pEP302 was prepared by the method of Furutani et al. (cf. Furutani, Y. et al., Nucleic Acids Res., Vol. 13, 5869, 1985) and it was digested with restriction enzymes HpaI and BamHI to isolate a large DNA fragment containing E. coli tryptophane operon promoter region and ampicillin resistance gene. This fragment was ligated by T4 DNA ligase with a chemically synthesized oligonucleotide adaptor of the following Formula [E].

```
5'-AACTAGTACGCAAGTTCACGTAAAAGGAGGTTT
    3'-TTGATCATGCGTTCAAGTGCATTTTCCTCCAAATT            [E]
```

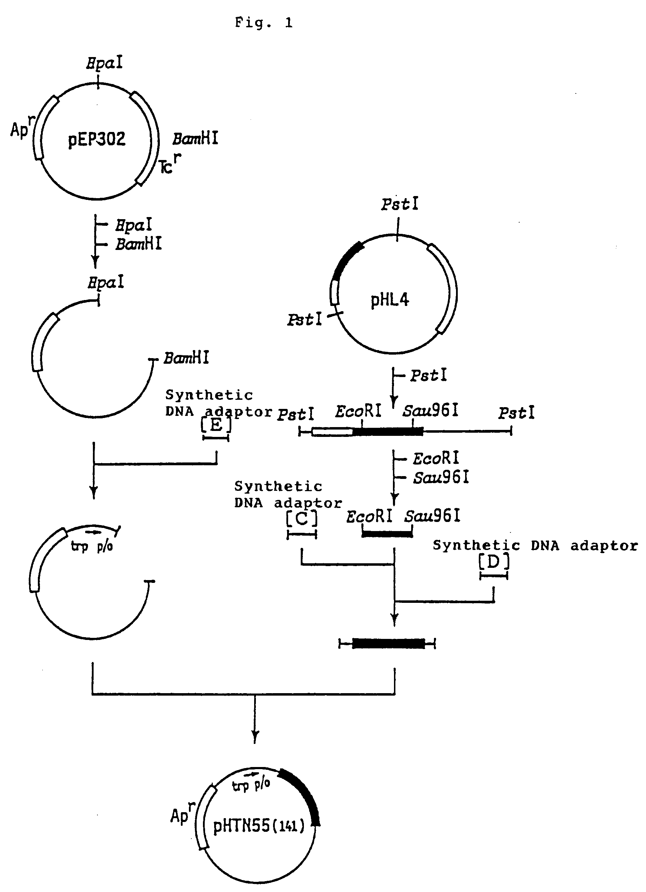

The DNA fragment was further ligated by T4 DNA ligase with the above IL1(141) fragment, and thereby, there was constructed an expression plasmid for producing TN-55(141) polypeptide (cf. the accompanying Fig. 1). This expression plasmid was designated as "pHTN55(141)".

(2) Production of TN-55(141) polypeptide:

A transformant was prepared by using the expression plasmid pHTN55(141) prepared in the above (1) in the same manner as described in Example 1, and the transformant was cultivated likewise, and TN-55-(141) polypeptide was isolated from the culture cells.

As to the purified TN-55(141) polypeptide, the amino acid sequence of the N-terminal region was analyzed by Edman degradation method. As a result, the polypeptide showed the following amino acid sequence of from the N-terminus to the 40th amino acid, which was well identical to the amino acid sequence of the formula [III] in Table 3 (X in Table 3 is Asn).

```
Met Arg Ile Ile Lys Tyr Glu Phe Ile Leu

Asn Asp Ala Leu Asn Gln Ser Ile Ile Arg

Ala Asn Asp Gln Tyr Leu Thr Ala Ala Ala

Leu His Asn Leu Asp Glu Ala Val Lys Phe
```

Besides, when it was digested with carboxypeptidase and the amino acid thereof was analyzed, there was detected leucine.

During the purification step of the TN-55(141) polypeptide, it was confirmed that a deamidated product having an acidic isoelectric point was present like in Example 2. When the amino acid sequence of the deamidated polypeptide was analyzed, it was confirmed that the 22nd amino acid at the N-terminus was exchanged for Asp.

The TN-55(141) polypeptide had an isoelectric point of 5.4 ± 0.2, and the deamidated polypeptide had an isoelectric point of 5.2 ± 0.2.

Reference Example 1

Construction of expression plasmid pHIPH383a:

A recombinant plasmid pHL4 inserted with a cloned cDNA encoding human interleukin 1 precursor polypeptide (cf. Table 8) was digested with a restriction enzyme PstI to cut out the cDNA region. The cloned cDNA was digested with restriction enzymes EcoRI and BstNI to isolate a DNA fragment which corresponds to the 398th to 808th bases in the nucleotide sequence in Table 8.

This DNA fragment was ligated by T4 DNA ligase with two kinds of chemically synthesized oligonucleotide adaptors having the following formula [F] and [G]. The DNA fragment thus obtained was designated as "SD-IL1 fragment". The chemically synthesized oligonucleotide adaptor of the formula [F] was a DNA adaptor prepared by ligating five kinds of DNA fragments of the following formula [a] to [e] in order (hereinafter referred to as "chemically synthesized DNA [F]").

```
5'-AACTAGTACGCAAGTTCAC
3'-TTGATCATGCGTTCAAGTGCATT            [a]

5'-GTAAAAGGAGGTTTAAA
   3'-TTCCTCCAAATTTAATAC            [b]

5'-TTATGTCATCACCTTTTAG
   3'-AGTAGTGGAAAATCGAAGG            [c]

5'-CTTCCTGAGCAATGTGAAATACAACTTTA
   3'-ACTCGTTACACTTTATGTTGAAATACTC       [d]

  5'-TGAGGATCATCAAATACG
   3'-CTAGTAGTTTATGCTTAA              [e]
```

The chemically synthesized oligonucleotide adaptor of the formula [G] has the following nucleotide sequence.

```
5'-AGGCGTGATGACTCGA
3'-CCGCACTACTGAGCTCTAG                 [G]
```

Separately, expression vector pEP302was digested with restriction enzymes HpaI and BamHI to isolate a large DNA fragment containing E. coli tryptophane operon promoter region and ampicillin resistance gene

EP 0 327 360 B1

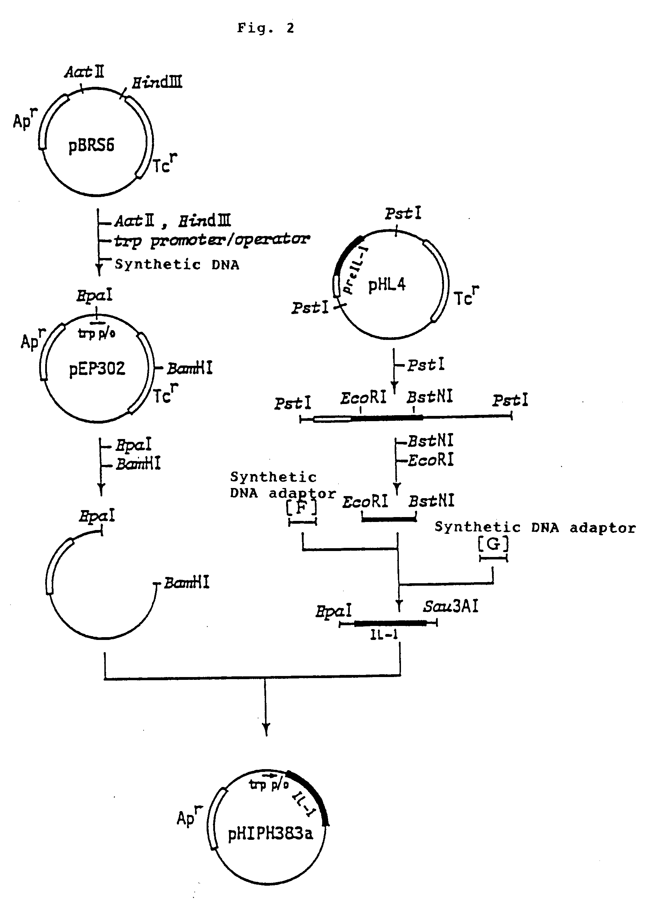

(hereinafter referred to as "vector fragment"). This vector fragment was ligated by T4 DNA ligase with the above SD-IL1 fragment, by which there was constructed an expression plasmid for producing human interleukin 1 polypeptide (cf. the accompanying Fig. 2).

This expression plasmid was designated as "pHIPH383a".

Reference Example 2

Construction of expression plasmid pHIP312EC:

Plasmid pBR322 was digested with restriction enzymes AvaI and PvuII to isolate a large DNA fragment (about 3.7 kbp). Both ends of this DNA fragment were digested to blunt ends with DNA polymerase I (klenow fragment) and dGTP, dATP, dCTP, dTTP and then ligated by using T4 DNA ligase to prepare a plasmid vector in which a region for controllig the copy number at around replication origin of pBR322 was deleted (hereinafter referred to as "pBRS6")

The plasmid vector pBRS6 was digested with a restriction enzyme EcoRI to give straight chain DNA, and both ends thereof were digested to blunt ends by treating with DNA polymerase I (Klenow fragment) and dGTP, dATP, dCTP, dTTP. To both ends of this DNA fragment was ligated by T4 DNA ligase an oligonucleotide linker of the following formula [H].

The chemically synthesized linker had the following nucleotide sequence.

5′-CCTCGAGG-3′     [H]

Subsequently, the DNA fragment thus obtained was digested with restriction enzymes PstI and XhoI to isolate a large DNA fragment containing tetracycline resistance gene. This DNA fragment was designated as "pBRS6-fragment".

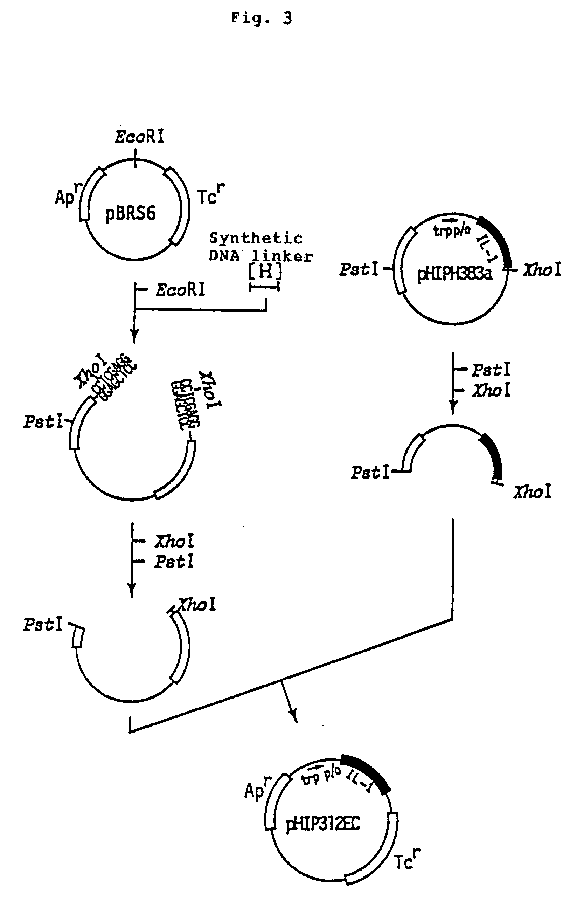

Separately, the recombinant plasmid pHIPH383a disclosed in Reference Example 1 was digested with restriction enzymes PstI and XhoI to isolate a DNA fragment containing a region encoding human interleukin 1 polypeptide. This DNA fragment was ligated with the above pBRS6-fragment, by which there was constructed an expression plasmid for producing human interleukin 1 polypeptide which contained tetracycline resistance gene (cf. the accompanying Fig. 3).

16

This expression plasmid was designated as "pHIP312EC".

### Table 8

Nucleotide sequence and amino acid sequence of cDNA encoding human interleukin 1 (α-type) precursor polypeptide:

```
     1                                          10
    Met Ala Lys Val Pro Asp Met Phe Glu Asp
    ATG GCC AAA GTT CCA GAC ATG TTT GAA GAC (30)

                                             20
    Leu Lys Asn Cys Tyr Ser Glu Asn Glu Glu
    CTG AAG AAC TGT TAC AGT GAA AAT GAA GAA (60)

                                             30
    Asp Ser Ser Ser Ile Asp His Leu Ser Leu
    GAC AGT TCC TCC ATT GAT CAT CTG TCT CTG (90)

                                             40
    Asn Gln Lys Ser Phe Tyr His Val Ser Tyr
    AAT CAG AAA TCC TTC TAT CAT GTA AGC TAT (120)

                                             50
    Gly Pro Leu His Glu Gly Cys Met Asp Gln
    GGC CCA CTC CAT GAA GGC TGC ATG GAT CAA (150)

                                             60
    Ser Val Ser Leu Ser Ile Ser Glu Thr Ser
    TCT GTG TCT CTG AGT ATC TCT GAA ACC TCT (180)

                                             70
    Lys Thr Ser Lys Leu Thr Phe Lys Glu Ser
    AAA ACA TCC AAG CTT ACC TTC AAG GAG AGC (210)

                                             80
    Met Val Val Val Ala Thr Asn Gly Lys Val
    ATG GTG GTA GTA GCA ACC AAC GGG AAG GTT (240)

                                             90
    Leu Lys Lys Arg Arg Leu Ser Leu Ser Gln
    CTG AAG AAG AGA CGG TTG AGT TTA AGC CAA (270)

                                            100
    Ser Ile Thr Asp Asp Asp Leu Glu Ala Ile
    TCC ATC ACT GAT GAT GAC CTG GAG GCC ATC (300)

                                            110
    Ala Asn Asp Ser Glu Glu Glu Ile Ile Lys
    GCC AAT GAC TCA GAG GAA GAA ATC ATC AAG (330)
```

```
                                        120
Pro Arg Ser Ser Pro Phe Ser Phe Leu Ser
CCT AGG TCA TCA CCT TTT AGC TTC CTG AGC (360)

                                        130
Asn Val Lys Tyr Asn Phe Met Arg Ile Ile
AAT GTG AAA TAC AAC TTT ATG AGG ATC ATC (390)

                                        140
Lys Tyr Glu Phe Ile Leu Asn Asp Ala Leu
AAA TAC GAA TTC ATC CTG AAT GAC GCC CTC (420)

                                        150
Asn Gln Ser Ile Ile Arg Ala Asn Asp Gln
AAT CAA AGT ATA ATT CGA GCC AAT GAT CAG (450)

                                        160
Tyr Leu Thr Ala Ala Ala Leu His Asn Leu
TAC CTC ACG GCT GCT GCA TTA CAT AAT CTG (480)

                                        170
Asp Glu Ala Val Lys Phe Asp Met Gly Ala
GAT GAA GCA GTG AAA TTT GAC ATG GGT GCT (510)

                                        180
Tyr Lys Ser Ser Lys Asp Asp Ala Lys Ile
TAT AAG TCA TCA AAG GAT GAT GCT AAA ATT (540)

                                        190
Thr Val Ile Leu Arg Ile Ser Lys Thr Gln
ACC GTG ATT CTA AGA ATC TCA AAA ACT CAA (570)

                                        200
Leu Tyr Val Thr Ala Gln Asp Glu Asp Gln
TTG TAT GTG ACT GCC CAA GAT GAA GAC CAA (600)

                                        210
Pro Val Leu Leu Lys Glu Met Pro Glu Ile
CCA GTG CTG CTG AAG GAG ATG CCT GAG ATA (630)

                                        220
Pro Lys Thr Ile Thr Gly Ser Glu Thr Asn
CCC AAA ACC ATC ACA GGT AGT GAG ACC AAC (660)

                                        230
Leu Leu Phe Phe Trp Glu Thr His Gly Thr
CTC CTC TTC TTC TGG GAA ACT CAC GGC ACT (690)

                                        240
Lys Asn Tyr Phe Thr Ser Val Ala His Pro
AAG AAC TAT TTC ACA TCA GTT GCC CAT CCA (720)
```

```
                                            250
Asn Leu Phe Ile Ala Thr Lys Gln Asp Tyr
AAC TTG TTT ATT GCC ACA AAG CAA GAC TAC (750)

                                            260
Trp Val Cys Leu Ala Gly Gly Pro Pro Ser
TGG GTG TGC TTG GCA GGG GGG CCA CCC TCT (780)

                                            270
Ile Thr Asp Phe Gln Ile Leu Glu Asn Gln
ATC ACT GAC TTT CAG ATA CTG GAA AAC CAG (810)

271
Ala***
GCGTAG
```

In the above table, the number means amino acid number, the parenthesized number means the nucleotide number, and *** means translation stop codon.

**Claims**
**Claims for the following Contracting States : AT, BE, CH, LI, DE, FR, GB, IT, LU, NL, SE**

1. A polypeptide of the interleukin 1α type characterized in that the interleukin 1α type polypeptide (1) is an interleukin 1α modified at least at position Y of the following formula (I) so as to be (a) essentially incapable of inducing the production of prostaglandin $E_2$ and (b) capable of activating lymphocytes and (2) has an amino acid sequence of the following formula [I] in which one to fourteen amino acid residues at the N-terminus and/or one to four amino acid residues at the C-terminus are optionally deleted:

EP 0 327 360 B1

```
Ser  W  Pro Phe Ser Phe Leu Ser Asn Val

Lys Tyr Asn Phe Met Arg Ile Ile Lys Tyr

Glu Phe Ile Leu Asn Asp Ala Leu Asn Gln

Ser Ile Ile Arg Ala  X  Asp Gln Tyr Leu

Thr Ala Ala Ala Leu His Asn Leu Asp Glu

Ala Val Lys Phe Asp Met Gly Ala Tyr Lys

Ser Ser Lys Asp Asp Ala Lys Ile Thr Val

Ile Leu Arg Ile Ser Lys Thr Gln Leu Tyr

Val Thr Ala Gln Asp Glu Asp Gln Pro Val

Leu Leu Lys Glu Met Pro Glu Ile Pro Lys

Thr Ile Thr Gly Ser Glu Thr Asn Leu Leu

Phe Phe Trp Glu Thr His Gly Thr Lys Asn

Tyr Phe Thr Ser Val Ala His Pro Asn Leu

Phe Ile Ala Thr Lys Gln Asp Tyr Trp Val

Cys Leu Ala Gly Gly Pro Pro Ser Ile Thr

 Y  Phe Gln Ile Leu Glu Asn Gln Ala
```

**Formula [I]**

in which W means Ser or Ala, X means Asn or Asp, and Y means an amino acid residue other than Asp.

2. A polypeptide according to claim 1, in which 14 amino acid residues at the N-terminus and 4 amino acids at the C-terminus are deleted.

3. A polypeptide according to claim 1, which has an amino acid sequence of formula (I) wherein Y is Tyr.

4. A polypeptide according to claim 3, wherein W is Ser and X is Asn.

5. A DNA encoding a polypeptide according to any one of claims 1 to 4.

6. A method for production of a polypeptide according to any one of claims 1 to 4, which comprises inserting the DNA encoding a polypeptide according to any one of claims 1 to 4 into an expression vector, transforming a host cell with the recombinant plasmid, cultivating the transformant, and collecting the resulting polypeptide from the cultivated cells by a conventional method.

**Claims for the following Contracting States : ES, GR**

1. A method for producing a polypeptide of the interleukin 1α type in which a recombinant host organism transformed with a DNA encoding the polypeptide is cultivated so as to express the polypeptide and the resulting polypeptide is collected from the cultivated cells by a conventional method, characterized

20

in that the interleukin 1α type polypeptide (1) is an interleukin 1α modified at least at position Y of the following formula (I) so as to be (a) essentially incapable of inducing the production of prostaglandin E$_2$ and (b) capable of activating lymphocytes and (2) has an amino acid sequence of the following formula [I] in which one to fourteen amino acid residues at the N-terminus and/or one to four amino acid residues at the C-terminus are optionally deleted:

```
Ser  W  Pro Phe Ser Phe Leu Ser Asn Val

Lys Tyr Asn Phe Met Arg Ile Ile Lys Tyr

Glu Phe Ile Leu Asn Asp Ala Leu Asn Gln

Ser Ile Ile Arg Ala  X  Asp Gln Tyr Leu

Thr Ala Ala Ala Leu His Asn Leu Asp Glu

Ala Val Lys Phe Asp Met Gly Ala Tyr Lys

Ser Ser Lys Asp Asp Ala Lys Ile Thr Val

Ile Leu Arg Ile Ser Lys Thr Gln Leu Tyr

Val Thr Ala Gln Asp Glu Asp Gln Pro Val

Leu Leu Lys Glu Met Pro Glu Ile Pro Lys

Thr Ile Thr Gly Ser Glu Thr Asn Leu Leu

Phe Phe Trp Glu Thr His Gly Thr Lys Asn

Tyr Phe Thr Ser Val Ala His Pro Asn Leu

Phe Ile Ala Thr Lys Gln Asp Tyr Trp Val


Cys Leu Ala Gly Gly Pro Pro Ser Ile Thr

 Y  Phe Gln Ile Leu Glu Asn Gln Ala
```

Formula [I]

in which W means Ser or Ala, X means Asn or Asp, and Y means an amino acid residue other than Asp.

2. A method according to claim 1, which comprises inserting the DNA encoding a polypeptide of the formula (I), defined in claim 1, into an expression vector, transforming a host cell with the recombinant plasmid, cultivating the transformant, and collecting the resulting polypeptide from the cultivated cells by a conventional method.

3. A method according to claim 1 or claim 2, in which 14 amino acid residues at the N-terminus and 4 amino acids at the C-terminus are deleted from the polypeptide.

4. A method according to claim 1 or claim 2, wherein the polypeptide has an amino acid sequence of formula (I) wherein Y is Tyr.

5. A method according to claim 4, wherein in the polypeptide W is Ser and X is Asn.

**Patentansprüche**
**Patentansprüche für folgende Vertragsstaaten : AT, BE, CH, LI, DE, FR, GB, IT, LU, NL, SE**

1. Polypeptid vom Interleukin 1α-Typ, dadurch gekennzeichnet, daß das Polypeptid vom Interleukin 1α-Typ (1) ein Interleukin 1α ist, das mindestens in Position Y der folgenden Formel (I) modifiziert ist, so daß es (a) im wesentlichen nicht die Produktion von Prostaglandin $E_2$ induzieren kann und (b) Lymphocyten aktivieren kann, und (2) eine Aminosäuresequenz der folgenden Formel aufweist, in welcher 1 bis 14 Aminosäuren am N-Terminus und/oder 1 bis 4 Aminosäuren am C-Terminus gegebenenfalls deletiert sind:

```
Ser   W   Pro Phe Ser Phe Leu Ser Asn Val

Lys Tyr Asn Phe Met Arg Ile Ile Lys Tyr

Glu Phe Ile Leu Asn Asp Ala Leu Asn Gln

Ser Ile Ile Arg Ala  X  Asp Gln Tyr Leu

Thr Ala Ala Ala Leu His Asn Leu Asp Glu

Ala Val Lys Phe Asp Met Gly Ala Tyr Lys

Ser Ser Lys Asp Asp Ala Lys Ile Thr Val

Ile Leu Arg Ile Ser Lys Thr Gln Leu Tyr

Val Thr Ala Gln Asp Glu Asp Gln Pro Val

Leu Leu Lys Glu Met Pro Glu Ile Pro Lys

Thr Ile Thr Gly Ser Glu Thr Asn Leu Leu

Phe Phe Trp Glu Thr His Gly Thr Lys Asn

Tyr Phe Thr Ser Val Ala His Pro Asn Leu

Phe Ile Ala Thr Lys Gln Asp Tyr Trp Val


Cys Leu Ala Gly Gly Pro Pro Ser Ile Thr

 Y  Phe Gln Ile Leu Glu Asn Gln Ala
```

Formel [I]

in der W Ser oder Ala bedeutet, X Asn oder Asp bedeutet und Y einen Aminosäurerest außer Asp bedeutet.

2. Polypeptid nach Anspruch 1, in dem 14 Aminosäurereste am N-Terminus und 4 Aminosäurereste am C-Terminus deletiert sind.

3. Polypeptid nach Anspruch 1, das eine Aminosäuresequenz der Formel (I) hat, wobei Y Tyr bedeutet.

4. Polypeptid nach Anspruch 3, wobei W Ser und X Asn bedeutet.

5. DNA, die ein Polypeptid nach einem der Ansprüche 1 bis 4 codiert.

6. Verfahren zur Produktion eines Polypeptids nach einem der Ansprüche 1 bis 4, umfassend die Insertion der ein Polypeptid nach einem der Ansprüche 1 bis 4 codierenden DNA in einen Expressionsvektor, Transformation einer Wirtszelle mit dem rekombinanten Plasmid, Züchtung der Transformante und Gewinnung des resultierenden Polypeptids aus den gezüchteten Zellen nach einem üblichen Verfahren.

**Patentansprüche für folgende Vertragsstaaten : ES, GR**

1. Verfahren zur Produktion eines Polypeptids vom Interleukin 1α-Typ, wobei ein rekombinanter Wirtsorganismus, der mit einer das Polypeptid codierenden DNA transformiert ist, gezüchtet wird, um das Polypeptid zu exprimieren, und das resultierende Polypeptid aus den gezüchteten Zellen nach einem üblichen Verfahren gewonnen wird, dadurch gekennzeichnet, daß das Polypeptid vom Interleukin 1α-Typ (1) ein Interleukin 1α ist, das mindestens in Position Y der folgenden Formel (I) modifiziert ist, so daß es (a) im wesentlichen nicht die Produktion von Prostaglandin E$_2$ induzieren kann und (b) Lymphocyten aktivieren kann, und (2) eine Aminosäuresequenz der folgenden Formel aufweist, in welcher 1 bis 14 Aminosäuren am N-Terminus und/oder 1 bis 4 Aminosäuren am C-Terminus gegebenenfalls deletiert sind:

```
Ser  W  Pro Phe Ser Phe Leu Ser Asn Val

Lys Tyr Asn Phe Met Arg Ile Ile Lys Tyr

Glu Phe Ile Leu Asn Asp Ala Leu Asn Gln

Ser Ile Ile Arg Ala  X  Asp Gln Tyr Leu

Thr Ala Ala Ala Leu His Asn Leu Asp Glu

Ala Val Lys Phe Asp Met Gly Ala Tyr Lys

Ser Ser Lys Asp Asp Ala Lys Ile Thr Val

Ile Leu Arg Ile Ser Lys Thr Gln Leu Tyr

Val Thr Ala Gln Asp Glu Asp Gln Pro Val

Leu Leu Lys Glu Met Pro Glu Ile Pro Lys

Thr Ile Thr Gly Ser Glu Thr Asn Leu Leu

Phe Phe Trp Glu Thr His Gly Thr Lys Asn
```

```
Tyr Phe Thr Ser Val Ala His Pro Asn Leu

Phe Ile Ala Thr Lys Gln Asp Tyr Trp Val

Cys Leu Ala Gly Gly Pro Pro Ser Ile Thr

 Y  Phe Gln Ile Leu Glu Asn Gln Ala
```

Formel [I]

in der W Ser oder Ala bedeutet, X Asn oder Asp bedeutet und Y einen Aminosäurerest außer Asp bedeutet.

2. Verfahren nach Anspruch 1, umfassend die Insertion der ein Polypeptid der Formel (I) gemäß der Definition in Anspruch 1 codierenden DNA in einen Expressionsvektor, Transformation einer Wirtszelle mit dem rekombinanten Plasmid, Züchtung der Transformante und Gewinnung des resultierenden Polypeptids aus den gezüchteten Zellen nach einem üblichen Verfahren.

3. Verfahren nach Anspruch 1 oder 2, in dem 14 Aminosäurereste am N-Terminus und 4 Aminosäurereste am C-Terminus des Polypeptids deletiert sind.

4. Verfahren nach Anspruch 1 oder 2, wobei das Polypeptid eine Aminosäuresequenz der Formel (I) hat, in der Y Tyr bedeutet.

5. Verfahren nach Anspruch 4, wobei im Polypeptid W Ser und X Asn bedeutet.

**Revendications**
**Revendications pour les Etats contractants suivants : AT, BE, CH, LI, DE, FR, GB, IT, LU, NL, SE**

1. Polypeptide de type interleukine 1$\alpha$, caractérisé en ce que le polypeptide de type interleukine 1$\alpha$
   (1) est une interleukine 1$\alpha$ modifiée au moins au niveau de la position Y de la formule (I) ci-dessous de façon à être (a) essentiellement incapable de déclencher la production de prostaglandine $E_2$ et (b) capable d'activer les lymphocytes, et
   (2) a une séquence d'aminoacides de la formule (I) ci-dessous dans laquelle un à quatorze résidus d'aminoacides à l'extrémité N-terminale et/ou un à quatre résidus d'aminoacides à l'extrémité C-terminale sont éventuellement supprimés:

```
Ser   W   Pro Phe Ser Phe Leu Ser Asn Val

Lys Tyr Asn Phe Met Arg Ile Ile Lys Tyr

Glu Phe Ile Leu Asn Asp Ala Leu Asn Gln

Ser Ile Ile Arg Ala   X  Asp Gln Tyr Leu

Thr Ala Ala Ala Leu His Asn Leu Asp Glu

Ala Val Lys Phe Asp Met Gly Ala Tyr Lys

Ser Ser Lys Asp Asp Ala Lys Ile Thr Val

Ile Leu Arg Ile Ser Lys Thr Gln Leu Tyr

Val Thr Ala Gln Asp Glu Asp Gln Pro Val

Leu Leu Lys Glu Met Pro Glu Ile Pro Lys

Thr Ile Thr Gly Ser Glu Thr Asn Leu Leu

Phe Phe Trp Glu Thr His Gly Thr Lys Asn

Tyr Phe Thr Ser Val Ala His Pro Asn Leu

Phe Ile Ala Thr Lys Gln Asp Tyr Trp Val

Cys Leu Ala Gly Gly Pro Pro Ser Ile Thr

 Y  Phe Gln Ile Leu Glu Asn Gln Ala
```

formule (I)

dans laquelle W signifie Ser ou Ala, X signifie Asn ou Asp, et Y représente un résidu d'aminoacide différent de Asp.

2. Polypeptide selon la revendication 1, dans lequel sont supprimés 14 résidus d'aminoacides à l'extrémité N-terminale et 4 aminoacides à l'extrémité C-terminale.

3. Polypeptide selon la revendication 1, qui a une séquence d'aminoacides de formule (I) dans laquelle Y est Tyr.

4. Polypeptide selon la revendication 3, dans lequel W est Ser et X est Asn.

5. ADN codant pour un polypeptide selon l'une quelconque des revendications 1 à 4.

6. Méthode de production d'un polypeptide selon l'une quelconque des revendications 1 à 4, dans laquelle on insère l'ADN codant pour un polypeptide selon l'une quelconque des revendications 1 à 4 dans un vecteur d'expression, on transforme une cellule hôte avec le plasmide recombiné, on cultive la cellule transformée, et on recueille par une méthode classique le polypeptide formé à partir des cellules cultivées.

**Revendications pour les Etats contractants suivants : ES, GR**

1. Méthode de production d'un polypeptide de type interleukine 1α, dans laquelle on cultive un organisme hôte recombiné transformé avec un ADN codant pour le polypeptide de façon à exprimer le polypeptide, et on recueille par une méthode classique le polypeptide formé à partir des cellules cultivées, caractérisée en ce que le polypeptide de type interleukine 1α

(1) est une interleukine 1α modifiée au moins au niveau de la position Y de la formule (I) ci-dessous de façon à être (a) essentiellement incapable de déclencher la production de prostaglandine $E_2$ et (b) capable d'activer les lymphocytes, et

(2) a une séquence d'aminoacides de la formule (I) ci-dessous dans laquelle un à quatorze résidus d'aminoacides à l'extrémité N-terminale et/ou un à quatre résidus d'aminoacides à l'extrémité C-terminale sont éventuellement supprimés:

```
Ser  W   Pro Phe Ser Phe Leu Ser Asn Val

Lys Tyr Asn Phe Met Arg Ile Ile Lys Tyr

Glu Phe Ile Leu Asn Asp Ala Leu Asn Gln

Ser Ile Ile Arg Ala  X  Asp Gln Tyr Leu

Thr Ala Ala Ala Leu His Asn Leu Asp Glu

Ala Val Lys Phe Asp Met Gly Ala Tyr Lys

Ser Ser Lys Asp Asp Ala Lys Ile Thr Val

Ile Leu Arg Ile Ser Lys Thr Gln Leu Tyr

Val Thr Ala Gln Asp Glu Asp Gln Pro Val

Leu Leu Lys Glu Met Pro Glu Ile Pro Lys

Thr Ile Thr Gly Ser Glu Thr Asn Leu Leu

Phe Phe Trp Glu Thr His Gly Thr Lys Asn

Tyr Phe Thr Ser Val Ala His Pro Asn Leu

Phe Ile Ala Thr Lys Gln Asp Tyr Trp Val

Cys Leu Ala Gly Gly Pro Pro Ser Ile Thr

 Y  Phe Gln Ile Leu Glu Asn Gln Ala
```

formule (I)

dans laquelle W signifie Ser ou Ala, X signifie Asn ou Asp, et Y représente un résidu d'aminoacide différent de Asp.

2. Méthode selon la revendication 1, dans laquelle on insère l'ADN codant pour un polypeptide de formule (I), défini dans la revendication 1, dans un vecteur d'expression, on transforme une cellule hôte avec le plasmide recombiné, on cultive la cellule transformée, et on recueille par une méthode classique le polypeptide formé à partir des cellules cultivées.

3. Méthode selon la revendication 1 ou la revendication 2, dans laquelle 14 résidus d'aminoacides à l'extrémité N-terminale et 4 aminoacides à l'extrémité C-terminale sont supprimés du polypeptide.

4. Méthode selon la revendication 1 ou la revendication 2, dans laquelle le polypeptide a une séquence d'aminoacides de formule (I) dans laquelle Y est Tyr.

26

**5.** Méthode selon la revendication 4, dans laquelle, dans le polypeptide, W est Ser et X est Asn.

EP 0 327 360 B1

Fig. 1

28

Fig. 2

Fig. 3